(19) Europäisches Patentamt
European Patent Office
Office européen des brevets

(11) Veröffentlichungsnummer: **0 231 744 B1**

(12) # EUROPÄISCHE PATENTSCHRIFT

(45) Veröffentlichungstag der Patentschrift: **30.05.90**

(51) Int. Cl.[5]: **C07D 403/10**, C07D 403/04, A61K 31/50

(21) Anmeldenummer: **87100041.0**

(22) Anmeldetag: **03.01.87**

(54) **Substituierte Pyrrol-1-ylphenyldihydropyridazinone, ihre Herstellung und Verwendung.**

(30) Priorität: **08.01.86 DE 3600275**

(43) Veröffentlichungstag der Anmeldung: **12.08.87 Patentblatt 87/33**

(45) Bekanntmachung des Hinweises auf die Patenterteilung: **30.05.90 Patentblatt 90/22**

(84) Benannte Vertragsstaaten: **AT BE CH DE ES FR GB IT LI NL SE**

(56) Entgegenhaltungen:
**EP-A- 0 075 436**
**EP-A- 0 167 995**
**DE-A- 2 304 977**

**CHEMICAL ABSTRACTS, Band 103, Nr. 19, 11. November 1985, Seite 708, Spalte 2, Zusammenfassungsnr. 160462a, Columbus, Ohio, US; I. SIRCAR et al.: "Cardiotonic agents. 2. Synthesis and structure-activity relationships of 4,5-dihydro-6-[4-(H-imidazol-1-yl)phenyl]-3(2H)-pyridazinones: a new class of positive inotropic agents"213 000**

(73) Patentinhaber: **BASF Aktiengesellschaft, Carl-Bosch-Strasse 38, D-6700 Ludwigshafen(DE)**

(72) Erfinder: **Geiss, Karl-Heinz, Dr., Kirchenstrasse 8, D-6711 Beindersheim(DE)**
Erfinder: **Schmied, Bernhard, Dr., Ringstrasse 1, D-6901 Dossenheim(DE)**
Erfinder: **Kropp, Rudolf, Sprottauer Strasse 2, D-6703 Limburgerhof(DE)**
Erfinder: **Baldinger, Verena, Dr., Schiffsgasse 6, D-6900 Heidelberg(DE)**
Erfinder: **Gries, Josef, Dr., Roemerweg 43, D-6706 Wachenheim(DE)**
Erfinder: **Lehmann, Hans Dieter, Prof. Dr., Im Hefen 15, D-6945 Hirschberg(DE)**
Erfinder: **Ruebsamen, Klaus, Dr., Erschigweg 19, D-6730 Neustadt(DE)**
Erfinder: **Traut, Martin, Dr., Mühltalstrasse 125, D-6900 Heidelberg(DE)**

EP 0 231 744 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Pyrrol-1-ylphenyldihydropyridazinone, Verfahren zu deren Herstellung sowie deren Verwendung bei der Bekämpfung von Krankheiten.

6-Aryldihydropyridazinone mit pharmakologischen Wirkungen sind bereits bekannt (vgl. z.B. J. Med. Chem. 17, 273 (1974), J. Het. Chem. 11, 755 (1974), J. Med. Chem. 26, 800 (1983). Eine Reihe von 6-Phenyl-dihydropyridazinonen, die die myokardiale Kontraktilität erhöhen und antihypertensiv wirken, sind in der EP-OS 75 436 und in J. Med. Chem. 28, 1405 - 1413 (1985) beschrieben.

Es wurde nun gefunden, daß substituierte Pyrrol-1-ylphenyldihydropyridazinone der Formel I

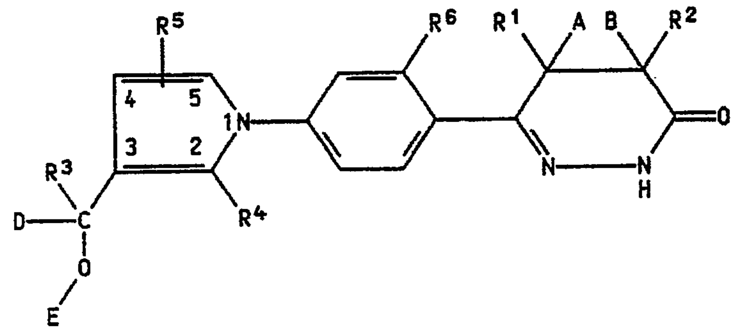

I,

worin

$R^1$ ein Wasserstoffatom, eine Methylgruppe oder eine Hydroxylmethylgruppe bedeutet,
$R^2$ ein Wasserstoffatom oder eine Methylgruppe bedeutet oder $R^1$ und $R^2$ zusammen den Rest $-(CH_2)_m-$, wobei m eine ganze Zahl von 1 bis 4 ist, bilden,
$R^3$, $R^4$ und $R^5$ unabhängig voneinander für Wasserstoffatome oder $C_{1-6}$-Alkylgruppen stehen,
$R^6$ ein Wasserstoffatom bedeutet oder zusammen mit $R^1$ den Rest $-(CH_2)_n-$, wobei n für 1, 2 oder 3 steht, oder den Rest -CH=CH- bildet,
A und B Wasserstoffatome bedeuten oder zusammen eine Bindung bilden,
D ein Wasserstoffatom, eine $C_{1-6}$-Alkylgruppe oder eine Phenylgruppe bedeutet,
E für ein Wasserstoffatom steht oder D und E zusammen eine Bindung bilden,
mit der Maßgabe, daß, falls $R^3$ ein Wasserstoffatom bedeutet, mindestens einer der Reste $R^4$, $R^5$, eine $C_{1-6}$-Alkylgruppe oder D eine $C_{1-6}$-Alkyl-oder Phenylgruppe bedeutet, wertvolle pharmakologische Eigenschaften besitzen.

Für die einzelnen Reste sind - unabhängig voneinander - die folgenden bevorzugten Bedeutungen zu nennen:

Bedeuten A und B Wasserstoffatome, so steht $R^1$ bevorzugt für ein Wasserstoffatom oder die Methylgruppe, insbesondere die Methylgruppe, und $R^2$ bevorzugt für ein Wasserstoffatom und $R^1$ und $R^2$ bilden zusammen eine Gruppe der Formel $-(CH_2)_m-$, in der n die Zahl 1 oder 2, insbesondere die Zahl 1, bedeutet.

Bilden A und B zusammen eine Bindung, so ist die bevorzugte Bedeutung für $R^1$ ein Wasserstoffatom und für $R^2$ ein Wasserstoffatom oder die Methylgruppe.

$R^3$ steht bevorzugt für ein Wasserstoffatom oder eine $C_{1-3}$-Alkylgruppe, insbesondere eine $C_{1-3}$-Alkylgruppe,
$R^4$ bedeutet bevorzugt ein Wasserstoffatom oder eine $C_{1-3}$-Alkylgruppe, insbesondere ein Wasserstoffatom,
$R^5$ bedeutet bevorzugt ein Wasserstoffatom oder eine $C_{1-3}$-Alkylgruppe, insbesondere eine $C_{1-3}$-Alkylgruppe,
$R^6$ steht bevorzugt für ein Wasserstoffatom. Bildet $R^6$ gemeinsam mit $R^1$ einen Rest $-(CH_2)_n-$ oder -CH=CH-, so steht n bevorzugt für 1 oder 2, insbesondere bedeuten $R^6$ und $R^1$ zusammen den Rest $-(CH_2)_2-$ oder -CH=CH-.
A und B bedeuten bevorzugt Wasserstoffatome.
D und E bilden bevorzugt eine gemeinsame Bindung. Steht D für ein Wasserstoffatom, eine $C_{1-6}$-Alkylgruppe oder eine Phenylgruppe, so ist die bevorzugte Bedeutung von D eine $C_{1-3}$-Alkylgruppe und die Phenylgruppe, insbesondere eine $C_{1-3}$-Alkylgruppe.

Die neuen Verbindungen der Formel I, in der D und E zusammen eine Bindung bilden, werden hergestellt, indem man p-Aminophenyldihydropyridazinone der Formel II

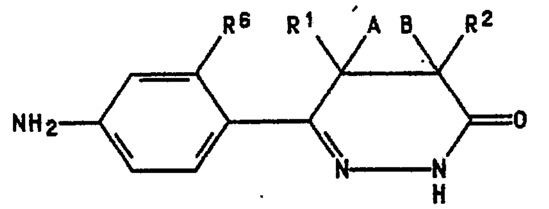

II,

worin $R^1$, $R^2$, $R^6$, A und B die für Formel I beschriebene Bedeutung besitzen, mit Tetrahydrofuranen der Formel III

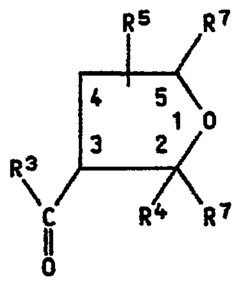

III,

worin $R^3$, $R^4$ und $R^5$ die in Formel I genannten Bedeutungen besitzen und $R^5$ an das Ringatom 4 oder 5 des Tetrahydrofuranringes gebunden ist und $R^7$ $C_{1-4}$-Alkoxy, $C_{1-4}$-Alkanoyloxyreste oder Chlor- oder Bromatome bedeuten, umsetzt und die so erhaltenen Verbindungen gegebenenfalls an der C=O-Gruppe hydriert oder mit einer Organometallverbindung F-Met, worin F eine $C_{1-6}$-Alkyl- oder eine Phenylgruppe und Met ein Alkalimetallatom oder der Rest MgX, worin X ein Chlor-, Brom- oder Iodatom ist, bedeuten, umsetzt.

Bevorzugte Reste für $R^7$ sind $C_{1-4}$-Alkoxyreste.

Falls $R^3$ und $R^4$ nicht zwei identische Reste ($R^a$ bzw. $R^b$) bedeuten, können die isomeren Produkte I a und I b gebildet werden,

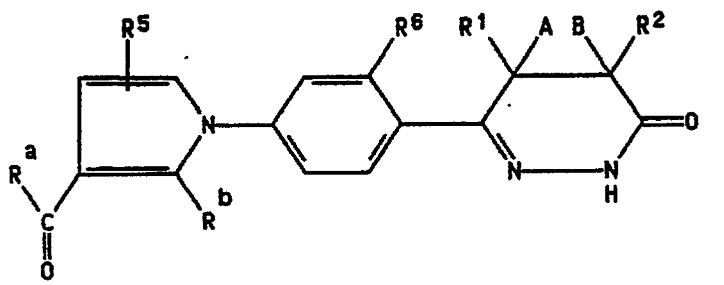

I a

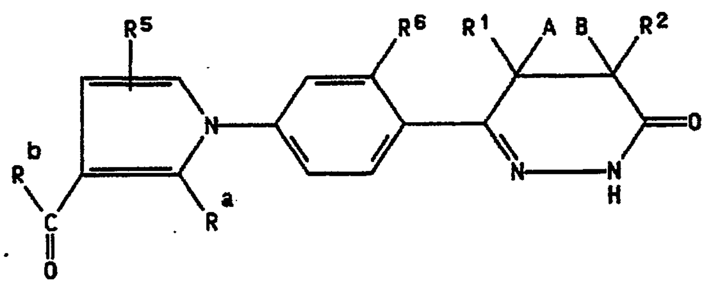

I b

worin die Reste $R^1$, $R^2$, $R^5$, $R^6$, A, B die für Formel I angegebene Bedeutung besitzen.

Die Herstellung der neuen Verbindungen der Formel I, in der D und E gemeinsam eine Bindung bilden, erfolgt unter sauren Bedingungen, entweder in einer niederen Alkancarbonsäure, wie Essigsäure, oder in einem organischen Lösungsmittel unter Zusatz einer Säure, wie einer anorganischen oder organischen Carbonsäure oder einer Sulfonsäure. Sie kann auch in Gegenwart eines sauer beladenen Ionenaustauschers durchgeführt werden. Die Säure wird in einer Menge von 0,01 bis 300 Molprozent eingesetzt, vorzugsweise in einer Menge von 0,1 bis 20 Molprozent. Die Umsetzung kann bei Normaldruck

oder erhöhtem Druck zwischen Raumtemperatur und der Rückflußtemperatur des eingesetzten Lösungsmittels durchgeführt werden. Üblicherweise werden Temperaturen zwischen 20 und 160°C, vorzugsweise 60 bis 120°C, angewendet. Geeignete organische Lösungsmittel sind aromatische Kohlenwasserstoffe - wie Benzol, Toluol, Ethylbenzol, Dichlorbenzol, o-, m-, p-Xylol, Methylnaphthalin -, aliphatische oder cycloaliphatische Kohlenwasserstoffe - wie Ligroin, Heptan, Cyclohexan -, Ether - wie Diethylether, Tetrahydrofuran -, Amide - wie N,N-Dimethylformamid, N,N-Dimethylacetamid, N-Methylpyrrolidon - oder Mischungen dieser Lösungsmittel.

Bevorzugt erfolgt die Herstellung in einer niederen Alkancarbonsäure, insbesondere in Essigsäure bei Temperaturen zwischen 60 und 120°C, insbesondere bei 70 bis 100°C.

Verbindungen der Formel I, in der D und E Wasserstoffatome bedeuten, werden durch Reduktion der Verbindungen der Formel I, in der D und E eine gemeinsame Bindung bilden, erhalten. Diese Reduktion kann beispielsweise mit Wasserstoff und Metallkatalysatoren, wie einem Platin-, Palladium-, Nickel- oder Kobaltkatalysator oder mit metallorganischen Hydriden wie Natriumborhydrid oder Lithiumaluminiumhydrid in einem unter den Reaktionsbedingungen inerten Lösungsmittel erhalten werden.

Die Umsetzung der Ketoverbindungen (I, D+E = gemeinsame Bindung) mit einer Organometallverbindung gelingt nach üblichen Methoden für Grignardreaktionen. Als Alkalimetalle kommen für den Rest F insbesondere Lithium und Natrium in Betracht.

Die Erfindung umfaßt alle enantiomeren, diastereomeren und tautomeren Formen der Verbindungen der Formel I.

Die Ausgangsprodukte der Formeln II und III sind bekannt, bzw. lassen sich analog zu literaturbekannten Methoden synthetisieren (vgl. zur Herstellung von Verbindungen der Formel III: Z.Chemie 13 (1973) 290-291 und Collect. Czech. Chem. Commun. 45 (1980) 888-891).

Die erfindungsgemäßen Verbindungen besitzen wertvolle pharmakologische Wirkungen. Insbesondere hemmen sie selektiv die Phosphodiesterase III, die cAMP-spezifisch ist und eine hohe Affinität (low Km) zu cAMP besitzt (vgl. Review zu Phosphodiesterase-Inhibitoren: J.Med.Chem. 28 (1985) 537-545).

Die erfindungsgemäßen Verbindungen hemmen die Thrombocytenaggregation und besitzen positiv inotrope, vasodilatatorische und coronardilatatorische Wirkungen. Außerdem senken sie den Blutdruck, hemmen die Magensäuresekretion und besitzen cytoprotektive Effekte auf die Magenschleimhaut. Sie eignen sich daher als Antihypertensiva und zur Behandlung von thrombotischen Erkrankungen, von Herzinsuffizienz und von Krankheitszuständen, die mit einer pathologisch gesteigerten Magensäuresekretion, wie Magen- und Duodenalulcera, einhergehen.

Die neuen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsform ab. In der Regel beträgt die tägliche Wirkstoffdosis zwischen etwa 0,1 bis 10, vorzugsweise 0,5 bis 5 mg bei parenteraler und 1 bis 100, vorzugsweise 5 bis 50 mg bei oraler Anwendung pro Patient und Tag.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Lösungen oder Suppositorien. Diese werden in üblicher Weise hergestellt. Die Wirkstoffe können dabei mit den gebräuchlichen galenischen Hilfsmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H.Sucker et al: Pharmazeutische Technologie, Thieme-Verlag, Stuttgart 1978). Die so erhaltenen Zubereitungen enthalten den Wirkstoff normalerweise in einer Menge von 0,1 bis 99 Gew.%.

Die Erfindung wird nachstehend anhand von Beispielen erläutert.

Beispiel 1A

6-[4-(4-Acetyl-2-methylpyrrol-1-yl)phenyl]-4,5-dihydropyridazin-3-on

Verbindung I, $R^1$=$R^2$=$R^4$=$R^6$=A=B=H, $R^3$ = $CH_3$, $R^5$ = 5-$CH_3$, D+E = gemeinsame Bindung.

Eine Lösung von 2,8g (15mmol) 6-(4-Aminophenyl)-4,5-dihydropyridazinon und 3,1 g (16,5 mmol) 2,5-Dimethyl-2,5-dimethoxytetrahydrofuran-3-carbaldehyd in 30 ml Essigsäure wurde 2 h bei 80°C gerührt. Anschließend wurde die Reaktionslösung in gesättigte Kaliumcarbonatlösung gegossen und mehrmals mit Methylenchlorid extrahiert. Nach Trocknen der organischen Phase über $Na_2SO_4$ und Abziehen des Lösungsmittels am Rotationsverdampfer wurde das Rohprodukt aus Dimethylformamid/Wasser umkristallisiert. Man erhielt 3,4 g 6-[4-(4-Acetyl-2-methylpyrrol-1-yl)phenyl]-4,5-dihydropyridazinon, Fp.188 - 189°C.

Beispiele 1B bis 1H

Analog Beispiel 1A wurden die Verbindungen der Beispiele 1B bis 1H hergestellt (Verbindungen der Formel I, $R^3$=$CH_3$, $R^4$=H, $R^5$=5-$CH_3$, D + E = gemeinsame Bindung):

| Beispiel | $R^6$ | $R^1$ | $R^2$ | A | B | Fp. °C |
|---|---|---|---|---|---|---|
| 1B | H | H | $CH_3$ | H | H | 159–160 |
| 1C | H | $CH_3$ | H | H | H | 202–203 |
| 1D | H | $-CH_2-$ | | H | H | 230–231 |
| 1E | H | $-CH_2-CH_2$ | | H | H | 208–209 |
| 1F | H | H | H | gemeinsame Bindung | | 231–232 |
| 1G | $-CH_2-CH_2$ | | H | H | H | 219–221 |
| 1H | H | H | $CH_3$ | H | H | 159–160 |

<u>Beispiele 1K bis 1S</u>

Analog Beispiel 1A werden die Verbindungen der Beispiele 1K bis 1S hergestellt (Verbindungen der Formel I, $R^3$=$CH_3$, $R^4$=H, $R^5$=5-$CH_3$, D + E = gemeinsame Bindung):

| Beispiel | $R^6$ | $R^1$ | $R^2$ | A | B |
|---|---|---|---|---|---|
| 1K | H | $CH_3$ | H | gemeinsame Bindung | |
| 1L | H | H | $CH_3$ | gemeinsame Bindung | |
| 1M | H | $CH_2OH$ | H | H | H |
| 1N | H | $-(CH_2)_4-$ | | H | H |
| 1O | $-CH_2$196 | | H | H | H |
| 1P | $-CH_2-$ | | H | gemeinsame Bindung | |
| 1Q | $-CH_2CH_2-$ | | H | gemeinsame Bindung<br>bei 228°C Zersetzung | |
| 1R | $-CH=CH-$ | | H | H | H |
| 1S | $-CH=CH-$ | | H | gemeinsame Bindung | |

<u>Beispiel 2A</u>

6-[4-(3-n-Butanoylpyrrol-1-yl)phenyl]-5-methyl-4,5-dihydropyridazin-3-on (2 $A_1$) und
6-[4-(3-Formyl-2-n-propylpyrrol-1-yl)-phenyl]-5-methyl-4,5-dihydropyridazin-3-on (2$A_2$)

Eine Mischung aus 5,0 g (24,6mmol)6-(p-Aminophenyl)-5-methyl-4,5-dihydropyridazinon und 6,5 g (32mmol) 3-n-Butanoyl-2,5-dimethoxytetrahydrofuran in 100 ml Essigsäure wurde 2h bei 80°C gerührt. Nach weiterer Zugabe von 2,0 g (10 mmol) 3-n-Butanoyl-2,5-dimethoxytetrahydrofuran wurde nochmals 2 h bei 80°C gerührt. Am Rotationsverdampfer wurde die Lösung eingeengt, anschließend wurde mit $K_2CO_3$-Lösung neutralisiert und mit $CH_2Cl_2$ extrahiert. Nach Trocknen der organischen Phase über $Na_2SO_4$ und Abziehen des Lösungsmittels wurde das Rohprodukt über 800g Kieselgel mit einer Methylenchlorid/Methanol-4:1-Mischung chromatographiert. Man erhielt nach Umkristallisation der entsprechenden produkthaltigen Fraktionen aus Essigester 0,8 g 6-[4-(3-Formyl-2-n-propylpyrrol-1-yl)-phenyl]-5-methyl-4,5-dihydropyridazin-3-on. Fp.148-149°C (2 $A_2$) sowie 1,45 g 6-[4-(3-n-Butanoylpyrrol-1-yl)-phenyl]-5-methyl-4,5-dihydropyridazin-3-on, Fp. 162-163°C (2 $A_1$).

<u>Beispiel 2B</u>

Analog Beispiel 2A wurden durch Umsetzung von p-Aminophenyl-5-methyl-4,5-dihydropyridazin-3-on mit 3-Propionyl-2,5-dimethoxytetrahydrofuran nach Säulenchromatographie über Aluminiumoxid mit Methylenchlorid/Essigester 4:1 als Laufmittel 6-[4(2-Ethyl-3-formylpyrrol-1-yl)phenyl]-5-methyl-4,5-dihydropyridazin-3-on, Fp. 192-193°C (2 $B_2$) und 6-[4-(3-Propionylpyrrol-1-yl)-phenyl]-5-methyl-4,5-dihydropyridazinon, Fp. (II $B_1$) erhalten.

<u>Beispiele 2$C_1$ - 2$F_2$</u>

Analog Beispiel 2A und 2B können durch Umsetzung von Aminophenyldihydropyridazinonen der Formel II mit 3-Alkanoyl-2,5-dimethoxytetrahydrofuranen der Formel III die Verbindungen der Beispiele 2$C_1$ - 2$F_2$ erhalten werden (Verbindungen I, D+E = gemeinsame Bindung $R^5$ = H, $R^6$ = H):

| Beispiel | $R^1$ | $R^2$ | $R^3$ | $R^4$ | A | B |
|---|---|---|---|---|---|---|
| $2C_1$ | $CH_3$ | H | $CH_3$ | H | H | H |
| $2C_2$ | $CH_3$ | H | H | $CH_3$ | H | H |
| $2D_1$ | H | H | $i{-}C_3H_7$ | H | H | H |
| $2D_2$ | H | H | H | $i{-}C_3H_7$ | H | H |
| $2E_1$ | | $-CH_2-$ | $C_2H_5$ | H | H | H |
| $2E_2$ | | $-CH_2-$ | H | $C_2H_5$ | H | H |
| $2F_1$ | $CH_3$ | H | $n{-}C_6H_{13}$ | H | H | H |
| $2F_2$ | $CH_3$ | H | H | $n{-}C_6H_{13}$ | H | H |

Beispiel 3

Analog Beispiel 1 A kann durch Umsetzung von 2,5-Dimethoxy-4-formyl-2-methyltetrahydrofuran mit 6-[p-Aminophenyl]-5-methyl-4,5-dihydropyridazin-3-on, 6-[4-(2-Methyl-4-formyl-pyrrol-1-yl)phenyl]-5-methyl-4,5-dihydropyridazinon erhalten werden.

Beispiele 4 A - 4 D

Analog Beispiel 1 A können durch Umsetzung von 2,5-Diethyl-2,5-dimethoxy- 3-formyl-tetrahydrofuran mit Aminophenyldihydropyridazinonen der Formel II die Verbindungen der Beispiele 4 A - 4 D erhalten werden. (Verbindungen der Formel I, D + E = gemeinsame Bindung, $R^3 = C_2H_5$, $R^4$ = H, $R^5 = 5\text{-}C_2H_5$, $R^6$ = H):

| Beispiel | $R^1$ | $R^2$ | A | B |
|---|---|---|---|---|
| 4A | H | H | H | H |
| 4B | $CH_3$ | H | H | H |
| 4C | | $-CH_2-$ | H | H |
| 4D | H | H | gemeinsame Bindung | |

Beispiel 5 A:

6-[4-(4-(Hydroxyeth-1-yl)-2-methyl-pyrrol-1-yl)phenyl-]-5-methyl-4,5-dihydropyridazin-3-on

Eine Lösung von 3,1 g (10 mmol) der Verbindung aus Beispiel 1 C in 100 ml Ethanol wurde mit 1,5 g (20 mmol) $NaBH_4$ über Nacht bei Raumtemperatur gerührt. Nach Einengen des Lösungsmittels am Rotationsverdampfer wurde mit Wasser versetzt und mit Essigester extrahiert. Nach Trocknen über $Na_2SO_4$ und Abziehen des Lösungsmittels am Rotationsverdampfer wurde das Rohprodukt aus Dimethylformamid umkristallisiert. Man erhielt 0,8 g 6-[4-(4-(Hydroxyeth-1-yl)-2-methyl-pyrrol-1-yl)phenyl]-5-methyl-4,5-dihydropyridazin-3-on als Diastereomerenmischung, Fp.75 - 78°C.

Beispiele 5 B - 5 H

Analog Beispiel 5 A können die Verbindungen der Beispiele 5 B - 5 H erhalten werden (Ausgangsprodukte für 5 B : 1 B; 5 C : 1 D; 5 D : 1 F; 5 E : 1 G; 5 F : 2 $A_2$; 5 G : 2 $A_1$; 5 H : 3). (Verbindungen der Formel I, D = E = H):

| Beispiel | $R^3$ | $R^4$ | $R^5$ | $R^6$ | $R^1$ | $R^2$ | A | B |
|---|---|---|---|---|---|---|---|---|
| 5B | $CH_3$ | H | $5-CH_3$ | H | H. | H | H | H |
| 5C | $CH_3$ | H | $5-CH_3$ | H | | $-CH_2-$ | H | H |
| 5D | $CH_3$ | H | $5-CH_3$ | H | H | H | gemeinsame Bindung | |
| 5E | $CH_3$ | H | $5-CH3$ | | $-CH_2-CH_2$ | H | H | H |
| 5F | H | $n-C_3H_7$ | H | H | $CH_3$ | H | H | H |
| 5G | $n-C_3H_7$ | H | H | H | $CH_3$ | H | H | H |
| 5H | H | H | $5-CH_3$ | H | $CH_3$ | H | H | H |

Beispiel 6 A

Eine Lösung von 2,8g (10 mmol) 6-[4-(3-Formylpyrrol-1-yl)phenyl]-5-methyl-4,5-dihydropyridazinon (siehe DE-OS 34 25 632) in 100 ml absolutem Tetrahydrofuran wurde bei -10°C mit 22 mmol Phenylmagnesiumbromid, als Lösung in Diethylether, versetzt und langsam auf Raumtemperatur erwärmt. Nach Rühren über Nacht wurde in Wasser gegossen, die Lösung mit Essigsäure auf pH 5 eingestellt und mit Essigester extrahiert. Nach Säulenchromatographie des Rohprodukts über Kieselgel mit $CH_2Cl_2/CH_3OH$ 98 : 2 - 9 : 1 erhielt man 6-[4-(3-(1-Phenyl-1-hydroxymethyl)-pyrrol-1-yl)phenyl]-5-methyl-4,5-dihydropyridazinon.

Beispiel 6 B

Durch Umsetzung von n-Hexylmagnesiumbromid mit 6-[4-(3-Formylpyrrol-1-yl)phenyl]-5-methyl-4,5-dihydropyridazinon analog Beispiel 6 A kann 6-[4-(3-(1-Hydroxyhept-1-yl)-pyrrol-1-yl)phenyl]-5-methyl-4,5-dihydropyridazinon erhalten werden.

Beispiel 6 C

Analog Beispiel 6 A kann durch Umsetzung von Methylmagnesiumiodid mit der Verbindung aus Beispiel 1 C 6-[4-(4-(2-Hydroxyprop-2-yl)-2-methyl-pyrrol-1-yl)phenyl]-5-methyl-4,5-dihydropyridazinon erhalten werden.

Beispiel 7

Analog Beispiel 1 A kann durch Umsetzung von 6-(4-Aminophenyl)-5-methyl-4,5-dihydropyridazinon mit 3-Formyl-2,5-dimethoxy-2,4-dimethyltetrahydrofuran 6-[4-(3-Acetyl-4-methyl-pyrrol-1-yl)-phenyl]-5-methyl-4,5-dihydropyridazinon erhalten werden.

**Patentansprüche für die Vertragsstaaten: BE, CH, DE, FR, GB, IT, LI, NL, SE**

1. Substituierte Pyrrol-1-ylphenyldihydropyridazinone der Formel I

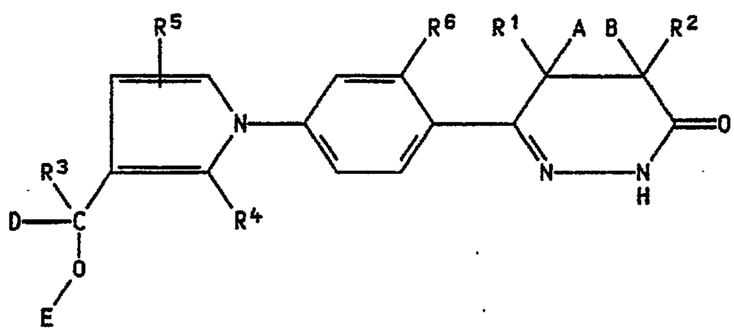

I,

worin
$R^1$ ein Wasserstoffatom, eine Methylgruppe oder eine Hydroxylmethylgruppe bedeutet,
$R^2$ ein Wasserstoffatom oder eine Methylgruppe bedeutet oder $R^1$ und $R^2$ zusammen den Rest $-(CH_2)_m-$, wobei m eine ganze Zahl von 1 bis 4 ist, bilden,
$R^3$, $R^4$ und $R^5$ unabhängig voneinander für Wasserstoffatome oder $C_{1-6}$-Alkylgruppen stehen,

$R^6$ ein Wasserstoffatom bedeutet oder zusammen mit $R^1$ den Rest $-(CH_2)_n-$, wobei n für 1, 2 oder 3 steht, oder den Rest -CH=CH-bildet,
A und B Wasserstoffatome bedeuten oder zusammen eine Bindung bilden,
D ein Wasserstoffatom, eine $C_{1-6}$-Alkylgruppe oder eine Phenylgruppe bedeutet,
E für ein Wasserstoffatom steht oder D und E zusammen eine Bindung bilden,
mit der Maßgabe, daß, falls $R^3$ ein Wasserstoffatom bedeutet, mindestens einer der Reste $R^4$, $R^5$, eine $C_{1-6}$-Alkylgruppe oder D eine $C_{1-6}$-Alkyl- oder Phenylgruppe bedeutet.

2. Verbindungen der Formel I gemäß Anspruch 1 zur Verwendung bei der Bekämpfung von Krankheiten.

3. Verwendung der Verbindungen der Formel I gemäß Anspruch 1 bei der Bekämpfung von hohem Blutdruck, thrombotischen Erkrankungen, Herzinsuffizienz und von Krankheitszuständen, die mit einer pathologisch gesteigerten Magensäuresekretion einhergehen.

4. Verfahren zur Herstellung der substituierten Pyrrol-1-ylphenyldihydropyridazinone der Formel I gemäß Anspruch 1, dadurch gekennzeichnet, daß man p-Aminophenyldihydropyridazinone der Formel II

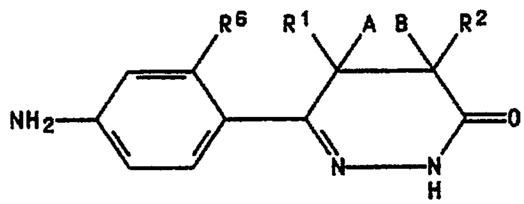

II,

worin $R^1$, $R^2$, $R^6$, A und B die für Formel I beschriebene Bedeutung besitzen, mit Tetrahydrofuranen der Formel III

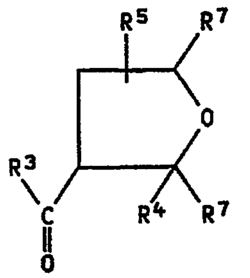

III,

worin $R^3$, $R^4$ und $R^5$ die in Formel I genannten Bedeutungen besitzen und $R^5$ an das Ringatom 4 oder 5 des Tetrahydrofuranringes gebunden ist und $R^7$ $C_{1-4}$-Alkoxy, $C_{1-4}$-Alkanoyloxyreste oder Chlor- oder Bromatome bedeuten, umsetzt und die so erhaltenen Verbindungen gegebenenfalls an der C=O-Gruppe hydriert oder mit einer Organometallverbindung F-Met, worin F eine $C_{1-6}$-Alkyl- oder eine Phenylgruppe und Met ein Alkalimetallatom oder den Rest MgX, worin X ein Chlor-, Brom-oder Iodatom ist, bedeuten, umsetzt.

**Patentanspruch für die Vertragsstaaten : AT, ES**

Verfahren zur Herstellung von substituierten Pyrrol-1-ylphenyldihydropyridazinonen der Formel I

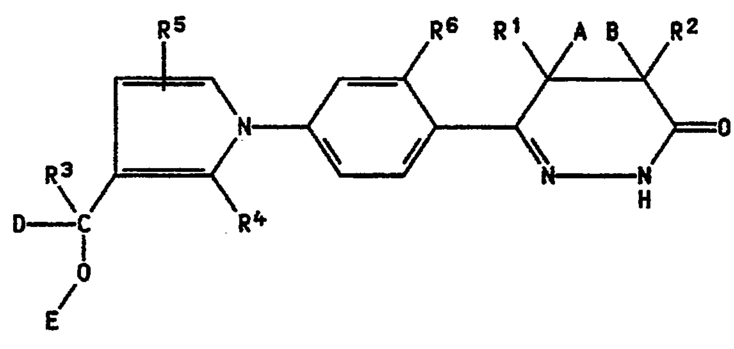

I,

worin
$R^1$ ein Wasserstoffatom, eine Methylgruppe oder eine Hydroxylmethylgruppe bedeutet,

$R^2$ ein Wasserstoffatom oder eine Methylgruppe bedeutet oder $R^1$ und $R^2$ zusammen den Rest $-(CH_2)_m-$, wobei m eine ganze Zahl von 1 bis 4 ist, bilden,
$R^3$, $R^4$ und $R^5$ unabhängig voneinander für Wasserstoffatome oder $C_{1-6}$-Alkylgruppen stehen,
$R^6$ ein Wasserstoffatom bedeutet oder zusammen mit $R^1$ den Rest $-(CH_2)_n-$, wobei n für 1, 2 oder 3 steht, oder den Rest -CH=CH-bildet,
A und B Wasserstoffatome bedeuten oder zusammen eine Bindung bilden,
D ein Wasserstoffatom, eine $C_{1-6}$-Alkylgruppe oder eine Phenylgruppe bedeutet,
E für ein Wasserstoffatom steht oder D und E zusammen eine Bindung bilden,
mit der Maßgabe, daß, falls $R^3$ ein Wasserstoffatom bedeutet, mindestens einer der Reste $R^4$, $R^5$, eine $C_{1-6}$-Alkylgruppe oder D eine $C_{1-6}$-Alkyl- oder Phenylgruppe bedeutet,
dadurch gekennzeichnet, daß man p-Aminophenyldihydropyridazinone der Formel II

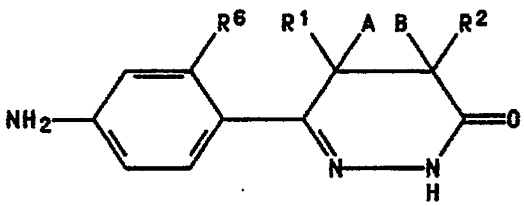

II,

worin $R^1$, $R^2$, $R^6$, A und B die für Formel I beschriebene Bedeutung besitzen, mit Tetrahydrofuranen der Formel III

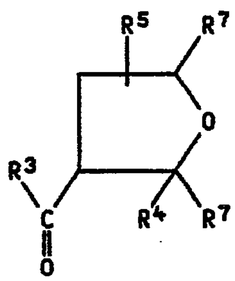

III,

worin $R^3$, $R^4$ und $R^5$ die in Formel I genannten Bedeutungen besitzen und $R^5$ an das Ringatom 4 oder 5 des Tetrahydrofuranringes gebunden ist und $R^7$ $C_{1-4}$-Alkoxy, $C_{1-4}$-Alkanoyloxyreste oder Chlor- oder Bromatome bedeuten, umsetzt und die so erhaltenen Verbindungen gegebenenfalls an der C=O-Gruppe hydriert oder mit einer Organometallverbindung F-Met, worin F eine $C_{1-6}$-Alkyl- oder eine Phenylgruppe und Met ein Alkalimetallatom oder den Rest MgX, worin X ein Chlor-, Brom-oder Iodatom ist, bedeuten, umsetzt.

**Claims for the Contracting States: BE, CH, DE, FR, GB, IT, LI, NL, SE**

1. A substituted pyrrol-1-ylphenyldihydropyridazinone of the furmula I

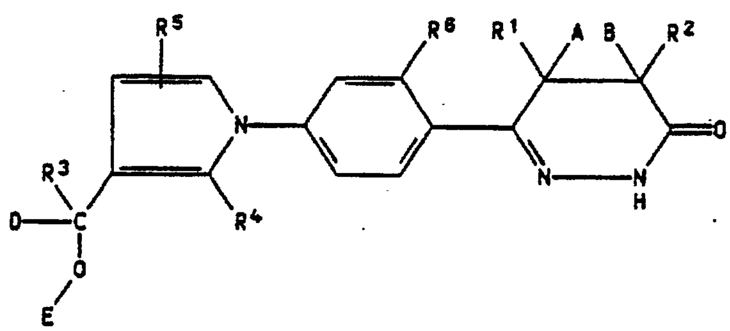

I

where $R^1$ is hydrogon, methyl or hydroxymethyl, $R^2$ is hydrogen or methyl, or $R^1$ and $R^2$ together form a radical $-(CH_2)_m-$, in which m is an integer from 1 to 4, $R^3$ $R^4$ and $R^5$ independently of one another are each hydrogen or $C_1$–$C_6$-alkyl, $R^6$ is hydrogen or, together with $R^1$, forms a radical $-(CH_2)_n-$, in which n

is 1, 2 or 3, or the radical –CH=CH–, A and B are each hydrogen or together form a bond, D is hydrogen, $C_1$–$C_6$-alkyl or phenyl, and E is hydrogen, or D and E together form a bond, with the proviso that, if $R^3$ is hydrogen, one or both of the radicals $R^4$ and $R^5$ are $C_1$–$C_6$-alkyl or D is $C_1$–$C_6$-alkyl or phenyl.

2. A compound of the formula I as claimed in claim 1 for use for the treatment of disorders.

3. Use of a compound of the formula I as claimed in claim 1 for the treatment of high blood pressure, thrombotic disorders, cardiac insufficiency and disorders accompanied by a pathological increase in the secretion of gastric acid.

4. A process for the preparation of a substituted pyrrol-1-ylphenyldihydropyridazinone of the formula I as claimed in claim 1, wherein a p-aminophenyldihydropyridazinone of the formula II

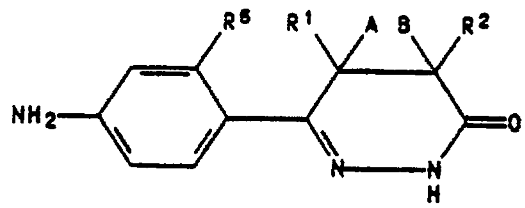

II

where $R^1$, $R^2$, $R^6$, A and B have the meanings described for formula I, is reacted with a tetrahydrofuran of the formula III

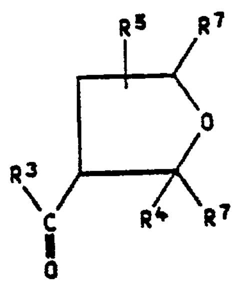

III

where $R^3$, $R^4$ and $R^5$ have the meanings stated in formula I, $R^5$ is bonded to ring atom 4 or 5 of the tetrahydrofuran ring and $R^7$ is $C_1$–$C_4$-alkoxy, $C_1$–$C_4$-alkanoyloxy, chlorine or bromine, and, if required, the compound thus obtained is hydrogenated at the C=O group or reacted with an organometallic compound F-Met, where F is $C_1$–$C_6$-alkyl or phenyl and Met is an alkali metal atom or a radical MgX, in which X is chlorine, bromine or iodine.

**Claim for the Contracting States: AS, ES**

A process for the preparation of a substituted pyrrol-1-ylphenyldihydropyridazinone of the formula I

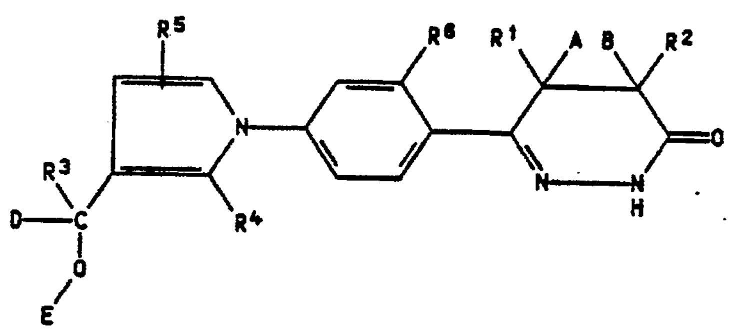

I

where $R^1$ is hydrogen, methyl or hydroxymethyl, $R^2$ is hydrogen or methyl, or $R^1$ and $R^2$ together form a radical $-(CH_2)_m-$, in which m is an integer from 1 to 4, $R^3$, $R^4$ and $R^5$ independently of one another are each hydrogen or $C_1$–$C_6$-alkyl, $R^6$ is hydrogen or, together with $R^1$, forms a radical $-(CH_2)_n-$,in which n is 1, 2 or 3, or the radical –CH=CH–, A and B are each hydrogen or together form a bond, D is hydrogen, $C_1$–$C_6$-alkyl or phenyl, and E is hydrogen, or D and E together form a bond, with the proviso that, if $R^3$ is hydrogen, one or both of the radicals $R^4$ and $R^5$ are $C_1$–$C_6$-alkyl or D is $C_1$–$C_6$-alkyl or phenyl, wherein a p-aminophenyldihydropyridazinone of the formula II

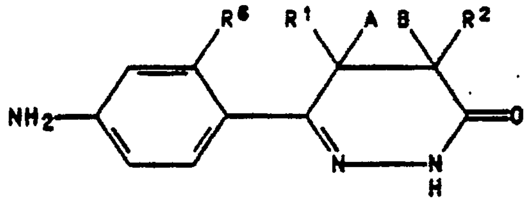

II

where $R^1$, $R^2$, $R^6$, A and B have the meanings described for formula I, is reacted with a tetrahydrofuran of the formula III

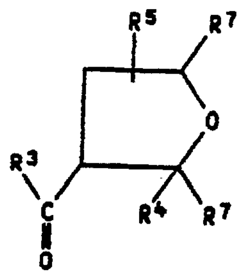

III

where $R^3$, $R^4$ and $R^5$ have the meanings stated in formula I, $R^5$ is bonded to ring atom 4 or 5 of the tetrahydrofuran ring and $R^7$ is $C_1$–$C_4$-alkoxy, $C_1$–$C_4$-alkanoyloxy, chlorine or bromine, and, if required, the compound thus obtained is hydrogenated at the C=O group or reacted with an organometallic compound F-Met, where F is $C_1$–$C_6$-alkyl or phenyl and Met is an alkali metal atom or a radical MgX, in which X is chlorine, bromine or iodine.

**Revendications pour les Etats contractants: BE, CH, DE, FR, GB, IT, LI, NL, SE**

1. Pyrrol-1-ylphényldihydropyridazinones substituées, de formule I

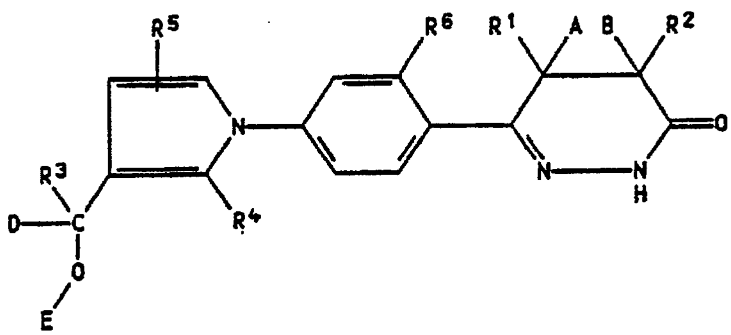

I,

dans laquelle
$R^1$ représente un atome d'hydrogène, un groupe méthyle ou un groupe hydroxyméthyle,
$R^2$ un atome d'hydrogène ou un groupe méthyle ou $R^1$ et $R^2$, ensemble, le reste $-(CH_2)_m-$, m étant un nombre entier de 1 à 4,
$R^3$, $R^4$ et $R^5$, indépendamment les uns des autres, étant mis pour des atomes d'hydrogène ou des groupes alkyle en $C_1$–$C_6$,
$R^6$ représente un atome d'hydrogène ou forme, ensemble avec $R^1$, le reste $-(CH_2)_n-$, n étant mis pour 1, 2 ou 3, ou le reste –CH=CH–,
A et B représentent des atomes d'hydrogène ou, ensemble, une liaison,
D un atome d'hydrogène, un groupe alkyle en $C_1$–$C_6$ ou un groupe phényle,
E est mis pour un atome d'hydrogène ou D et E forment, ensemble, une liaison, étant entendu que lorsque $R^3$ représente un atome d'hydrogène, au moins un des restes $R^4$, $R^5$ représente un groupe alkyle en $C_{1-6}$ ou D un groupe alkyle en $C_{1-6}$ ou un groupe phényle.

2. Composés de formule I selon la revendication 1 pour l'utilisation dans la lutte contre des maladies.

3. Utilisation des composés de formule I selon la revendication 1 pour la lutte contre l'hypertension, des maladies dues à des thromboses, l'insuffisance cardiaque et des états maladies accompagnant une sécrétion d'acide gastrique pathologiquement augmentée.

4. Procédé de préparation des pyrrol-1-ylphényldihydropyridazinones substituées de formule I, selon la revendication 1, caractérisé par le fait que l'on fait réagir des p-aminophényldihydropyridazionones de formule II

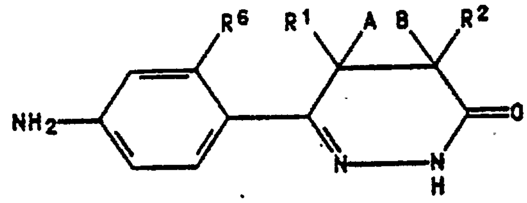

II,

où $R^1$, $R^2$, $R^6$, A et B ont les significations données pour la formule I, avec des tétrahydrofurannes de formule III

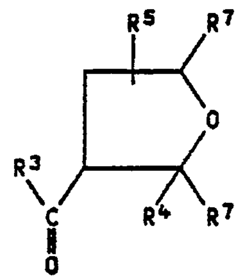

III,

où $R^3$, $R^4$ et $R^5$ ont les significations données pour la formule I, $R^5$ étant lié à l'atome cyclique 4 ou 5 du cycle tétrahydrofuranne, et $R^7$ représente alcoxy en $C_{1-4}$, des restes alcanoyloxy en $C_{1-4}$ ou des atomes de chlore ou de brome, et on hydrogène éventuellement les composés ainsi obtenus sur le groupe C=O ou on les fait réagir avec un composé organométallique F-mét ou F représente un groupe alkyle en $C_{1-6}$ ou un groupe phényle et mét un atome de métal alcalin ou le reste MgX où X désigne un atome de chlore, brome ou iode.

**Revendication pour les Etats contractants: AT, ES**

Procédé de préparation de pyrrol-1-ylphényldihydropyridazinones substituées, de formule I

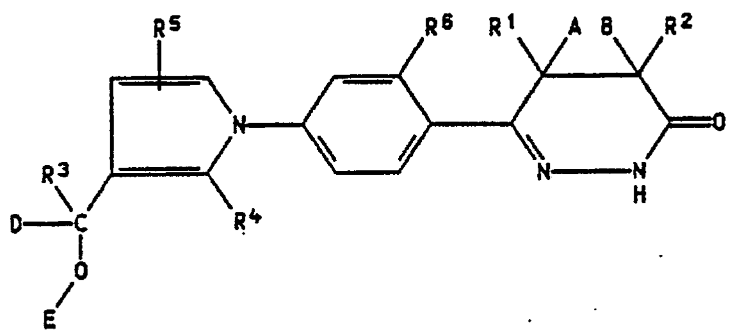

I,

dans laquelle
$R^1$ représente un atome d'hydrogène, un groupe méthyle ou un groupe hydroxyméthyle,
$R^2$ un atome d'hydrogène ou un groupe méthyle ou $R^1$ et $R^2$, ensemble, le reste $-(CH_2)_m-$, m étant un nombre entier de 1 à 4,
$R^3$, $R^4$ et $R^5$, indépendamment les uns des autres, étant mis pour des atomes d'hydrogène ou des groupes alkyle en $C_1$–$C_6$,
$R^6$ représente un atome d'hydrogène ou forme, ensemble avec $R^1$, le reste $-(CH_2)_n-$, n étant mis pour 1, 2 ou 3, ou le reste –CH=CH–,
A et B représentent des atomes d'hydrogène ou, ensemble, une liaison,
D un atome d'hydrogène, un groupe alkyle en $C_1$–$C_6$ ou un groupe phényle,
E est mis pour un atome d'hydrogène ou D et E forment, ensemble, une liaison,
étant entendu que lorsque $R^3$ représente un atome d'hydrogène; au moins un des restes $R^4$, $R^5$ représente un groupe alkyle en $C_{1-6}$ ou D un groupe alkyle en $C_{1-6}$ ou un groupe phényle caractérisé par le fait que l'un fait réagir des p-aminophényldihydropyridazinones de formule II

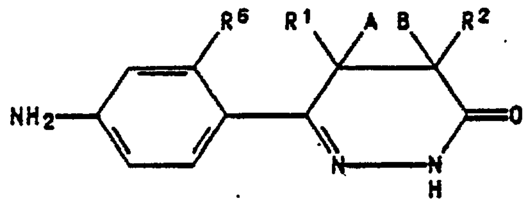

II,

où $R^1$, $R^2$, $R^6$, A et B ont les significations données pour la formule I, avec des tétrahydrofurannes de formule III

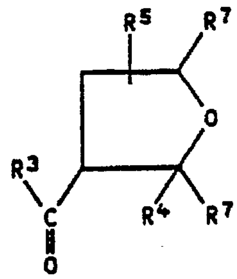

III,

où $R^3$, $R^4$ et $R^5$ ont les significations données pour la formule I, $R^5$ étant lié à l'atome cyclique 4 ou 5 du cycle tétrahydrofuranne, et $R^7$ représente alcoxy en $C_{1-4}$, des restes alcanoyloxy en $C_{1-4}$ ou des atomes de chlore ou de brome, et on hydrogène éventuellement les composés ainsi obtenus sur le groupe C=O ou on les fait réagir avec un composé organométallique F-mét ou F représente un groupe alkyle en $C_{1-6}$ ou un groupe phényle et mét un atome de métal alcalin ou le reste MgX où X désigne un atome de chlore, brome ou iode.